# EUROPÄISCHE PATENTANMELDUNG

(11) **EP 2 027 855 A1**
(43) Veröffentlichungstag der Anmeldung: **25.02.2009**
(21) Anmeldenummer: 07016642.6
(22) Anmeldetag: 24.08.2007
(51) Int. Cl.: A61K 31/195, A61K 31/495, A61K 31/505, A61K 31/519, A61K 31/565, A61K 31/57, A61P 15/02, A61P 15/12, A61P 15/18, A61P 19/10, A61P 43/00

(54) **Verwendung von Gestagenen in Kombination mit (6S)5-Methyltetrahydrofolat zur Therapie der Endometriose mit gleichzeitiger Verminderung von Therapie-Nebenwirkungen sowie der Verminderung des Risikos angeborener Fehlbildungen bei Eintritt einer Gravidität**

(71) Anmelder: Bayer Schering Pharma AG, 13353 Berlin (DE)
(72) Erfinder: Seitz, Christian, 15738 Zeuthen (DE); Waßerfall, Annemarie, 13503 Berlin (DE); Diefenbach, Konstanze, 10117 Berlin (DE); King, Christina, 10437 Berlin (DE); Zimmermann, Holger, 14612 Falkensee (DE)

(57) **Zusammenfassung**

Antiandrogene Gestagene in einer täglichen Dosiseinheit, welche maximal dem Zweifachen der Ovulationshemmdosis entspricht werden in Kombination mit (6S)-5-Methyltetrahydrofolat zur Herstellung pharmazeutischer Präparate zur Therapie der Endometriose mit gleichzeitiger Verminderung von Therapie-Nebenwirkungen, wie dem negativen Einfluss auf die Knochendichte/den Knochenstoffwechsel und dem Osteoporose-Risikio, sowie, bei Eintritt einer Gravidität, der Verminderung des Risikos angeborener Fehlbildungen, wie Neuralrohrdefekten, Lippen-Kiefer-Gaumenspalten, und Schwangerschaftskomplikationen, wie Plazentaablösung und Frühgeburtlichkeit verwendet.

Die Erfindung ist zur Langzeitanwendung geeignet.

## Beschreibung

Die Erfindung betrifft die Verwendung von Gestagenen in Kombination mit (6S)-5-Methyltetrahydrofolat, wobei die tägliche Dosis des Gestagens maximal dem Zweifachen der Ovulationshemmdosis entspricht, zur Herstellung pharmazeutischer Präparate zur Therapie der Endometriose mit gleichzeitiger Verminderung von Therapie-Nebenwirkungen, wie dem negativen Einfluss auf die Knochendichte/den Knochenstoffwechsel und auf das Osteoporose-isikio, sowie, bei Eintritt einer Gravidität, der Verminderung des Risikos angeborener Fehlbildungen, wie Neuralrohrdefekten, Lippen-Kiefer-Gaumenspalten, und Schwangerschaftskomplikationen, wie Plazentaablösung und Frühgeburtlichkeit. Die Erfindung ist zur Langzeitanwendung geeignet.

### Stand der Technik

Die Endometriose ist eine chronische, gynäkologische Erkrankung, die primär bei 5-20% der Frauen im gebärfähigen Alter vorkommt. In der Fachliteratur wird die Endometriose definiert als das Vorkommen von Endometrium oder endometriumähnlichem Gewebe außerhalb des Cavum uteri. Typische Beschwerdebilder der Endometrioseerkrankung sind die Dysmenorrhoe, Dyspareunie und Schmerzen beim Stuhlgang. Endometriose-Patientinnen klagen häufig über Schmerzen im Beckenbereich. Unterleibsschmerzen, die in der 2. Zyklushälfte auftreten, gefolgt von einer schmerzhaften Regelblutung und anschließender Beschwerdefreiheit bis Mitte des folgenden Zyklus lassen häufig an eine Endometrioseerkrankung denken, aber auch dauerhafte Schmerzen sind nicht selten. Allerdings haben ca. 30- 40 % der an Endometriose Erkrankten keine Beschwerden. Die Erkrankung wird dann nur zufällig im Zusammenhang mit anderen diagnostischen Maßnahmen festgestellt. In ca. 50-60% wird die Diagnose "Erkrankung an Endometriose" als zufällige Diagnose bei der Abklärung einer Sterilität gestellt.

Aus der Fach- und Patentliteratur ist bekannt, Endometriose medikamentös mit Danazol, einem Derivat von 17α-Ethinyltestosteron, GnRH-Agonisten, Gestagen/Estrogen-Kombinationen oder Gestagen-Monopräparaten zu behandeln.

US 6,569,845 offenbart die Behandlung von angiogenen Erkrankungen mit Dienogest in einer täglichen Dosis von 0,5 bis 10 mg. Entsprechende, als Beispiele ausgewiesene pharmazeutische Zusammensetzungen, die man weitläufig auch zur Behandlung von Endometriose einsetzen könnte, besitzen einen Dienogest-Gehalt von 400 mg bis 2 g.

Moore, C. *et al*., The treatment of endometriosis, Drugs of Today 1999, 35 (Suppl C): 41-52 untersuchten in klinischen Studien die Wirksamkeit von Dienogest bei der Behandlung von Endometriose vergleichsweise zum Behandlungsregime mit Danazol oder GnRH-Agonisten. Den Endometriose Betroffenen wurden 24 Wochen 2 mg Dienogest pro Tag verabreicht. Das Ergebnis der Behandlung ist vergleichbar mit dem Ergebnis einer Behandlung mit Danazol oder GnRH-Agonisten. Bis zu 90 % der Betroffenen berichteten über irreguläre Blutungen, jedoch keine berichtete über unerträgliche Blutungen. Die Wirksamkeit der Standardbehandlung mit Danazol wird durch signifikante androgene Effekte geschmälert, während GnRH-Agonisten mit "menopausalen Symptomen" in Zusammenhang stehen.

Schweppe, K.-W, Stellenwert der Gestagene, Zentralbl Gynakol 2003, 125: 276-280 erklärt, dass bei kontinuierlicher oraler Gestagenbehandlung (beispielsweise mit Medroxyprogesteronacetat, Dienogest, Dydrogesteron, Lynestrenol in einer täglichen Dosierung von 5 mg bis 20 mg, als niedrig dosiert bezeichnet und eingestuft als wirksames Behandlungsprinzip bei Endometriose bedingten Symptomen) niedrige Estrogenspiegel zu verzeichnen sind. Es resultieren häufig Schmier- und Zwischenblutungen. Dies zwingt zur Dosiserhöhung und/oder Estrogenzugabe. Rezidivraten liegen langfristig über 50%.

Eine Sicherheitsinformation von 2005 zu einem Medroxyprogesteronacetat-Produkt zeigt auf, dass Gestagen-Monopräparate besonders bei Langzeitbehandlung negative Wirkung auf die Knochendichte ausüben können.

In Kombination mit Estrogenen dagegen üben einige Gestagene einen positiven Einfluss auf den Knochenstoffwechsel aus.

Eine weitere Sicherheitsinformation, NDA 21-584, FDA 22.03.2005, zu deposubQ provera 104^{™} (Medroxyprogesteronacetat i.m. - 104 mg/0.65 ml), verweist darauf, dass Frauen, welche dieses Präparat verwenden, einen Knochenmineraldichteverlust erleiden, der sich mit der Dauer der Verwendung des Präparates vergrößert und nicht mehr komplett reversibel ist.

Knauthe, R und Habenicht U.F., Levonorgestrel has benefical effects, Exp Clin Endocrinol diabetes 106 (1998) Suppl 1: 37 zeigen bereits 1998 auf, dass dabei die partielle androgene Wirkung eines Gestagens (Levonorgestrel) und nicht die gestagene Aktivität ausschlaggebend ist für diesen positiven Einfluss auf den Knochenstoffwechsel. Auch Kuhl, H., Klimakterium, Postmenopause und Hormonsubstitution, 3. Aufl., Bremen. UNI-MED, 2006, 117 betont, dass bestimmte Gestagene über ihre androgene Partialwirkung wirksam werden. Ferner erklärt Kuhl, dass Androgene die positive Wirkung der Estrogene auf die Knochendichte erheblich verstärken.

Die Osteoporose wird In der Fachliteratur als nicht-rückgängig zu machender Knochenabbau mit erhöhter Knochenbrüchigkeit aufgezeigt. Mehr als 5 Millionen Menschen in Deutschland leiden an Osteoporose. Frauen sind häufiger betroffen als Männer. Die Osteoporose ist ein schmerzloser, schleichender Vorgang, bei dem nicht nur die Menge der Knochensubstanz abnimmt, sondern auch die "Architektur" des Knochens so verändert wird, dass dieser den normalen Belastungen nicht mehr standhält. Eine häufige Osteopotosefolge ist die Schenkelhalsfraktur oder die äußerst schmerzhaften Wirbelkörperbrüche. Trotz Behandlung ist das Risiko weiterer Knochenbrüche sehr hoch. Die Basismedikation bei Osteoporose ist die Kombination von Kalzium mit Vitamin D. mittel der ersten Wahl für eine Osteoporose-Therapie sind die Bisphosphonate Alendronat und Risedronat sowie der selektive Estrogen-Rezeptor-modulator Raloxifen.

BRÖLL, H. et al erklärt in "Konsensus-Statement: Therapie der postmenopausalen Osteoporose, J.Miner.Stoffwechs. 1/2007, 45-48, dass als Abbruchgründe einer Therapie mit oralen Bisphosphonaten, die Nebenwirkungen an erster Stelle stehen. Zu den Nebenwirkungen der bezeichneten Bisphosphonate gehören Bauchschmerzen, grippeähnliches Syndrom, Verstopfung, peripheres Ödem, Tinnitus und Bronchitis. Nachteilig erweist sich ebenfalls, dass wegen der Gefahr von Schleimhautentzündungen Bisphosphonate streng nach Vorschrift einzunehmen sind; Morgens nüchtern im Stehen mit einem großen Glas Wasser . Nach Einnahme sollte man sich mindestens eine halbe Stunde nicht hinlegen.

Die Nachteile von Raloxifen sind in der Literatur mit möglichen Hitzewallungen, Wadenkrämpfe und Ödeme beschrieben.

Estrogenhaltige Präparate werden in der Hormonersatztherapie zum Erhalt der Knochenmasse angewendet. Eine estrogen-haltige Therapie ist ebenfalls mit gewissen Risiken verbunden. Sie sollte zur Vermeidung von Gebärmutterschleimhautkrebs nur bei Frauen mit intakter Gebärmutter angewendet werden.

Es ist auch bekannt, dass Endometriose mit Subfertilität assoziiert ist und häufig während der Abklärung eines unerfüllten Kinderwunsches diagnostiziert wird. Ein Therapieziel bei Endometriose ist daher häufig auch, die

Wahrscheinlichkeit für eine Schwangerschaft zu erhöhen.

Ferner ist bekannt, dass ein unzureichender Folatstatus in der Schwangerschaft zu angeborenen Fehlbildungen, wie beispielsweise angeborene Herzfehler, angeborene Fehlbildungen der Harnwege, eine akute lymphoblastische Leukämie, Lippen-, Kiefer- und Gaumenspalten oder Fehlbildungen des Zentralnervensystems, wie Neuralrohrdeffekte (Spina bifida oder Anencephalie) führen können.

Die Deutsche Gesellschaft für Ernährung e.V. empfiehlt deshalb als Tagesdosis grundsätzlich 400 µg Folsäure, für Schwangere 600 µg und für stillende Mütter 600 µg. Dies ist eine globale Aussage. Mangel an Vitamin B₁₂ und Folatmangel weisen im Blutbild identische Veränderungen auf. Durch Verabreichung von Folat/Folsäure kann der Folatmangel kompensiert werden, jedoch der Mangel an Vitamin B₁₂ wird nicht angezeigt. Es ist damit die Gefahr eines maskierten Vitamin-B₁₂-Mangels gegeben.

Folsäure, auch Pteroyl-mono-glutaminsäure, N-(4-(((2-Amino-1,4-dihydro-4-oxo-6-pteridinyl)methyl)-amino)benzoyl)glutaminsäure (Summenformel: C₁₉H₁₉N₇)O₆), Folinsäure genannt, ist ein hitze- und lichtempfindliches, wasserlösliches Vitamin aus dem Vitamin-B-Komplex (Vitamin B₉).

Es ist bekannt, dass in der Nahrung Folate überwiegend als Pteroylpolyglutamate vorliegen. Diese werden nach Nahrungsaufnahme zunächst in den Mukosazellen zu Pteroylmonoglutamaten hydrolisiert, dann im Darm durch aktiven Transport hauptsächlich resorbiert.

In der Leber werden die überwiegend nicht-methylierten Folate in methylierte Folate umgewandelt und hauptsächlich als 5-Methyltetrahydrofolat (5-MTHF) an Albumin und α-Makroglobulin gebunden an die Zellen weiter transportiert, dort aufgenommen, demethyliert und in die Polyglutamatform umgewandelt.

An der Demethylierung sind die Aminosäure Homocystein sowie ein Enzym, welches Vitamin B₁₂ als Coenzym benötigt, beteiligt.

Ferner ist bekannt, dass Verluste am Folatgehalt von Lebensmitteln durch die Zubereitung (Kochen) sowie die Lagerung entstehen können. Weiterhin ist bekannt, dass auf die menschliche Haut treffende intensive UV-Strahlung die Folsäure im Körper reduziert. Hellhäutige Menschen sind dabei besonders betroffen.

Bei unzureichender Versorgung mit Folat und/oder Vitamin B₁₂ wird der Homocysteinstoffwechsel behindert, als Folge kann die Konzentration von Homocystein im Blut ansteigen. Die Konzentration an Homocystein im Blut kann demnach als ein Indikator für den Folatgehalt herangezogen werden.

Der Zustand der Hyperhomocysteinämie wird nach Malinow, MR et al, Homocyst(e)ine, diet, and cardiovascular disease, Statement for healthcare professionals from the Nutrition Committee, American Heart Association. Circulation 99, 178-182, 1999, durch folgende Konzentration im Plasma definiert: 16 - 30µmol/l (moderat); 31 - 100µmol/l (mittel); > 100µmol/l (schwer). Eine Konzentration über 10µmol/l wird als kritisch angesehen und ab 12µmol/l besteht Handlungsbedarf.

Neben dem Mangel an Folat und dem Vitamin B₁₂ können aber auch Enzymdefekte den Anstieg der Homocysteinkonzentration bewirken. Der Zusammenhang zwischen erhöhten Homocysteinkonzentrationen im Blut und

Gefäßerkrankungen, beispielsweise auch als Risikofaktor für Herz-Kreislauf-Erkrankungen wird seit einiger Zeit diskutiert.

Ebenso wird diskutiert, ob Folsäure/Folat wegen seiner Bedeutung für die DNA-Methylierung und die DNA-Strangstabilität vor malignen Erkrankungen schützen kann.

Die Patentschrift EP 0 898 965 beansprucht die Verwendung von 5-Methyl-(6S)-tetrahydrofolsäure oder deren pharmazeutisch verträgliche Salze zur Vorbeugung von Neuralrohrdefekten.

Die Patentschrift EP 1 044 975 offenbart kristalline Salze der 5-Methyl-(6R,S)-, -(6S)-und-(6R)-tetrahydrofolsäure und der Verwendung als Bestandteil Nahrungsmittelergänzungsstoff.

### Darstellung der Erfindung

Aufgabe der Erfindung ist es, eine Möglichkeit zu finden, Endometriose-Patientinnen vor einem Osteoporoserisiko zu schützen, die Endometriose zu vermindern und das Erreichen eines Zieles der Endometrioseverminderung, nämlich den Eintritt einer Schwangerschaft, ohne Nebenwirkungen zu realisieren.

Es wurde nun gefunden, dass die Aufgabe erfindungsgemäß gelöst wird durch die Verwendung von Gestagenen in Kombination mit (6S)-5-Methyltetrahydrofolat (Metafolin), wobei die tägliche Dosis des Gestagens maximal dem Zweifachen und mindestens der Ovulationshemmdosis entspricht, gemeinsam oder getrennt mit einem oder mehreren pharmazeutisch annehmbaren Hilfsstoffen/Trägern zur Herstellung pharmazeutischer Präparate.

Es wurde gefunden, dass neben der Minderung der Endometriose überraschenderweise keine negative Beeinflussung des Knochenstoffwechsels erfolgt, so dass keine Abnahme/Verringerung der Knochendichte zu verzeichnen ist, kein Osteoporoserisiko besteht und dass das Risiko angeborener Fehlbildungen, wie Neuralrohrdeffekten und Lippen-Kiefer-Gaumenspalten, und Schwangerschaftskomplikationen, wie Plazentaablösung und Frühgeburtlichkeit bei Eintritt einer Gravidität ebenfalls vermindert werden kann.

Gleichwohl gelingt es mit der erfindungsgemäßen Verwendung des pharmazeutischen Präparates überraschenderweise, die von den konventionellen Arzneimitteln zur Behandlung der Endometriose bekannten Nebenwirkungen, z.B. Hitzewallungen, Änderung des Lipidprofils im erträglichen Maße zu halten.

Erfindungsgemäß sind die verwendeten Gestagene mit antiandrogener Wirksamkeit Dienogest, Cyproteronacetat oder Chlormadinonacetat.

Die tägliche Dosis an den Gestagenen kann bis maximal das Zweifache der Ovulationshemmdosis betragen.

Die tägliche Dosis an Dienogest beträgt 1 mg bis zu maximal 2 mg. Auch Cyproteronacetat oder Chlormadinonacetat werden erfindungsgemäß in einer täglichen Dosierung bis zum Zweifachen der Ovulationshemmdosis eingesetzt. Die Ovulationshemmdosis von Cyproteronacetat beträgt 1 mg, die von Chlormadinonacetat 1.7 mg.

Auch wird die Aufgabe erfindungsgemäß durch die Verwendung von 0.1 bis 10 mg (6S)-5-Methyltetrahydrofolat als tägliche Dosiseinheit, bevorzugt 0.4 bis 1 mg (6S)-5-Methyltetrahydrofolat , besonders bevorzugt 451 µg (6S)-5-Methyltetrahydrofolat.

Die erfindungsgemäße Verwendung realisiert auch die Herstellung pharmazeutischer Präparate mit einer kontinuierlichen Verabreichung der Dosierungsform für die Dauer von mindestens 169 Tagen oder 25 Wochen bis mehreren Jahren, vorzugsweise mehr als 2 Jahren und ist daher überraschenderweise für die Langzeitapplikation geeignet.

Für die erfindungsgemäße Verwendung können Tabletten, Kapseln, Dragees, Wafer, Transdermalen-Therapie-Systemen, Ampullen, Suppositorien, Gelen, Salben, Implantaten, Vaginalringen oder Nasenspray eingesetzt werden.

Dabei ist die von den nichtoralen Formen des pharmazeutischen Präparates, wie Transdermales-Therapie-System, Ampulle, Suppositorie, Gel, Salbe, Implantat, Vaginalring oder Nasenspray abgegebene tägliche Gestagendosis äquivalent zum Ein- bis Zweifachen der Ovulationshemmdosis betragende täglichen Dosiseinheit bei den oralen Formen bzw. maximal äquivalent zur Wirksamkeit von 2 mg Dienogest.

Weiterhin wird die Aufgabe durch ein Verfahren zur Herstellung eines pharmazeutischen Präparates zur Endometriose-Therapie mit gleichzeitiger Verminderung von Therapie-Nebenwirkungen, wie dem negativen Einfluss auf die Knochendichte/den Knochenstoffwechsel und auf das Osteoporose-Risikio, sowie, bei Eintritt einer Gravidität, der Verminderung des Risikos angeborener Fehlbildungen und Schwangerschaftskomplikationen wie Neuralrohrdefekten, Lippen-Kiefer-Gaumenspalten, Plazentaablösung und Frühgeburtlichkeit, enthaltend eine Kombination von Gestagenen mit (6S)-5-Methyltetrahydrofolat und, gemeinsam oder getrennt, einen oder mehrere pharmazeutisch annehmbare Hilfsstoffe/Träger, wobei die tägliche Dosis des Gestagens mindestens der Ovulationshemmdosis bis maximal dem Zweifachen der Ovulationshemmdosis entspricht.

Weitere Ausführungsformen des durch das erfindungsgemäße Verfahren hergestellte pharmazeutische Präparates entsprechen den Ansprüchen 11,12, 13, 14, 15, 16, 17 und 18.

### Ausführungsbeispiele

### Beispiel 1

Es werden Tabletten mit folgender Zusammensetzung hergestellt:

### Kern:

| | |
|---|---|
| Dienogest | 2.000 mg |
| Metafolin | 0.451 mg |
| Laktose-Monohydrat | 46.349 mg |
| Microcrystalline Cellulose | 24.800 mg |
| Hydroxypropylcellulose | 1.600 mg |
| Croscarmellose | 3.200 mg |
| Magnesiumstearat | 1.600 mg |

Alle Substanzen werden in geeigneter Weise gemischt, tablettiert und gegebenenfalls befilmt.

### Beispiel 2

Es werden Tabletten mit folgender Zusammensetzung hergestellt:

| | |
|---|---|
| Dienogest, micronisiert min, 99% ≤ 20 pm, 100 % < 30 µm | 2.000 mg |
| Lactose-Monohydrat | 62.800 mg |
| Microkristalline Cellulose | 18.000 mg |
| Kartoffelstärke | 36.000 mg |
| Povidon K 25 | 8.100 mg |
| Magnesiumstearat | 1.350 mg |
| Talkum | 4.050 mg |
| Crospovidon | 2.700 mg |

Dienogest wird micronisiert mit einer mittleren Teilchengröße von 20 µm eingesetzt und mit Lactose-Monohydrat, Microkristalliner Cellulose und Kartoffelstärke vermischt. Das Povidon K 25 wird während der Granulierung eingesprüht. Nach Trocknen und Zumischen von Talkum, Crospovidon und Magnesiumstearat wird die Mischung aus den Substanzen zu Tabletten mit einem Durchmesser von 7 mm und einer Masse von 135 mg gepresst.

451 µg (6S)-5-Methyltetrahydrofolat werden separat mit einem oder mehreren pharmazeutisch annehmbaren Hilfsstoffen/Trägern nach bekannten Methoden verarbeitet und gleichzeitig mit der Dienogest-Formulierung verabreicht.

### Beispiel 3

In einer klinischen Studie wurden 252 Frauen mit laparoskopisch diagnostizierter Endometriose über einen Zeitraum von 6 Monaten entweder mit dem GnRH-Agonisten Leuprorelin Acetat (LA) 3,75 mg s.c. alle 4 Wochen oder mit 2mg/d oral des Gestagens Dienogest (DNG) behandelt. 128 Patientinnen wurden in die LH-Gruppe und 124 Patientinnen in die DNG-Gruppe randomisiert. Die Wirksamkeit der jeweiligen Therapie wurde u.a. mittels einer von der Patientin auszufüllenden Schmerzskale (Visual Analogue Scale, VAS) untersucht. Am Ende der Behandlung zeigte sich in beiden Vergleichsgruppen eine ähnliche Reduktion der Schmerzen im Vergleich zum Studienbeginn (-47,5 mm für DNG; -46,0 mm für LA). Die statistische Analyse zeigte die Nicht-Unterlegenheit von DNG gegenüber LA. Gleichzeitig wurde in einer Subpopulation nachgewiesen, dass in der Dienogestgruppe kein Knochendichteabfall erfolgte wogegen in der LA-Gruppe die Knochendichte um 4 % abnahm.

## Patentansprüche

1. Verwendung von Gestagenen in Kombination mit (6S)-5-Methyltetrahydrofolat, wobei die tägliche Dosis des Gestagens mindestens der Ovulationshemmdosis bis maximal dem Zweifachen der Ovulationshemmdosis entspricht, zur Herstellung pharmazeutischer Präparate zur Therapie der Endometriose mit gleichzeitiger Verminderung von Therapie-Nebenwirkungen, wie dem negativen Einfluss auf die Knochendichte/den Knochenstoffwechsel und dem Osteoporose-Risikio, sowie der Verminderung des Risikos angeborener Fehlbildungen und Schwangerschaftskomplikationen bei Eintritt einer Gravidität.

2. Verwendung von Gestagenen und (6S)-5-Methyltetrahydrofolat nach Anspruch 1, wobei die angeborenen Fehlbildungen und Schwangerschaftskomplikationen bei Eintritt einer Gravidität Neuralrohrdefekte, Lippen-Kiefer-Gaumenspalten, Plazentaablösung und Frühgeburtlichkeit sind.

3. Verwendung von Gestagenen und (6S)-5-Methyltetrahydrofolat nach Anspruch 1 oder 2, **dadurch gekennzeichnet, dass** die Gestagenkomponente 17α-Cyanomethyl-17-β-hydroxyestra-4,9-dien-3on (Dienogest), Cyproteronacetat oder Chlormadinonacetat ist.

4. Verwendung von Gestagenen und (6S)-5-Methyltetrahydrofolat nach Anspruch 1, 2 oder 3, **dadurch gekennzeichnet, dass** die tägliche Gestagendosis 1 bis 2 mg Dienogest oder eine äquivalente Menge an Cyproteronacetat oder Chlormadinonacetat beträgt.

5. Verwendung von Gestagenen und (6S)-5-Methyltetrahydrofolat nach Anspruch 1, 2, 3 oder 4, **dadurch gekennzeichnet, dass** die tägliche Dosis (6S)-5-Methyl-tetrahydrofolat 0.1 bis 10 mg beträgt.

6. Verwendung von Gestagenen und (6S)-5-Methyltetrahydrofolat nach Anspruch 1, 2, 3, 4 oder 5, **dadurch gekennzeichnet, dass** die tägliche Dosis (6S)-5-Methyltetrahydrofolat 0.4 bis 1 mg beträgt.

7. Verwendung von Gestagenen und (6S)-5-Methyltetrahydrofolat nach Anspruch 1, 2, 3, 4, 5 oder 6, **dadurch gekennzeichnet, dass** die tägliche Dosis des (6S)-5-Methyltetrahydrofolat 451 µg des Kalziumsalzes der (6S)-5-Methyltetrahydrofolsäure beträgt

8. Verwendung von Gestagenen und (6S)-5-Methyltetrahydrofolat nach Anspruch 1, 2, 3, 4, 5, 6 oder 7 zur Herstellung pharmazeutischer Präparate mit kontinuierlicher Verabreichung für die Dauer von mindestens 169 Tagen oder 25 Wochen bis mehreren Jahren, vorzugsweise mehr als 2 Jahren, zur Vermeidung von Nebenwirkungen, wie dem negativen Einfluss auf die Knochendichte/den Knochenstoffwechsel und das Osteoporose-Risikio, sowie der Verminderung des Risikos angeborener Fehlbildungen bei Eintritt einer Gravidität während oder nach der Endometriose-Therapie.

9. Verwendung von Gestagenen und (6S)-5-Methyltetrahydrofolat nach Anspruch 1, 2, 3, 4, 5, 6, 7 oder 8, **dadurch gekennzeichnet, dass** das pharmazeutische Präparat in Form von Tabletten, Kapseln, Dragees, Wafer, Transdermalen-Therapie-Systemen, Ampullen, Suppositorien, Gelen, Salben, Implantaten, Vaginalringen oder Nasenspray vorliegt.

10. Verfahren zur Herstellung eines pharmazeutischen Präparates zur Endometriose-Therapie mit gleichzeitiger Verminderung von Therapie-Nebenwirkungen, wie dem negativen Einfluss auf die Knochendichte/den Knochenstoffwechsel und dem Osteoporose-Risiko, sowie der Verminderung des Risikos angeborener Fehlbildungen und Schwangerschaftskomplikationen bei Eintritt einer Gravidität nach Anspruch 1,
**dadurch gekennzeichnet, dass** eine Kombination von Gestagenen mit (6S)-5-Methyltetrahydrofolat verwendet wird, wobei die tägliche Dosis des Gestagens mindestens der Ovulationshemmdosis bis maximal dem Zweifachen der Ovulationshemmdosis entspricht.

11. Verfahren nach Anspruch 10,
**dadurch gekennzeichnet, dass** die angeborenen Fehlbildungen und Schwangerschaftskomplikationen bei Eintritt einer Gravidität Neuralrohrdeffekte, Lippen-Kiefer-Gaumenspalten, Plazentaablösung und Frühgeburtlichkeit sind.

12. Verfahren nach Anspruch 10 oder 11,
**dadurch gekennzeichnet, dass** die Gestagenkomponente 17α-Cyanomethyl-17-β-hydroxyestra-4,9-dien-3on (Dienogest), Cyproteronacetat oder Chlormadinonacetat ist.

13. Verfahren nach Anspruch 10, 11 oder 12,
**dadurch gekennzeichnet, dass** die tägliche Dosis 1 bis 2 mg Dienogest oder eine äquivalente Menge an Cyproteronacetat oder Chlormadinonacetat und (6S)-5-Methyltetrahydrofolat beträgt.

14. Verfahren nach Anspruch 10, 11, 12 oder 13,
**dadurch gekennzeichnet, dass** die tägliche Dosis 0.1 bis 10 mg (6S)-5-Methyltetrahydrofolat beträgt.

15. Verfahren nach Anspruch 10, 11, 12, 13 oder 14,
**dadurch gekennzeichnet, dass** die tägliche Dosis 0.4 bis 1 mg (6S)-5-Methyltetrahydrofolat beträgt.

16. Verfahren nach Anspruch 10, 11, 12, 13, 14 oder 15,
**dadurch gekennzeichnet, dass** die tägliche Dosis 451 µg des Kalziumsalzes der (6S)-5-Methyltetrahydrofolsäure beträgt.

17. Verfahren nach Anspruch 10, 11, 12, 13, 14, 15 oder 16,
mit einer kontinuierlichen Verabreichung für die Dauer von mindestens 169 Tagen oder 25 Wochen bis mehreren Jahren, vorzugsweise mehr als 2 Jahren.

18. Verfahren nach Anspruch 10, 11, 12, 13, 14, 15, 16 oder 17,
**dadurch gekennzeichnet, dass** das pharmazeutische Präparat in Form von Tabletten, Kapseln, Dragees, Wafer, Transdermalen-Therapie-Systemen, Ampullen, Suppositorien, Gelen, Salben, Implantaten, Vaginalringen oder Nasenspray vorliegt.
